# NEUE EUROPÄISCHE PATENTSCHRIFT

(11) **EP 0 327 628 B2**
(45) Veröffentlichungstag und Bekanntmachung des Hinweises auf die Entscheidung über den Einspruch: **19.11.1997**
(45) Hinweis auf die Patenterteilung: 29.01.1992
(21) Anmeldenummer: 88907037.1
(22) Anmeldetag: 29.07.1988
(51) Int. Cl.: A61C 7/00, B23K 26/18, B21C 51/00

(54) **ORTHODONTISCHES HILFSTEIL MIT EINER MARKIERUNG UND VERFAHREN ZUR HERSTELLUNG DESSELBEN**
ORTHODONTIC ACCESSORY WITH MARKING AND PROCESS FOR MANUFACTURING SAME
ACCESSOIRE ORTHODONTIQUE REPERE ET PROCEDE POUR SA FABRICATION

(30) Priorität: 14.08.1987 DE 3727102
(43) Veröffentlichungstag der Anmeldung: 16.08.1989
(73) Patentinhaber: DENTAURUM J.P. WINKELSTROETER KG, 75228 Ispringen (DE)
(72) Erfinder: RÖHLCKE, Friedrich-Wilhelm, D-7539 Kämpfelbach-Bilfingen (DE); SERNETZ, Friedrich, D-7530 Pforzheim (DE)
(74) Vertreter: Hoeger, Stellrecht & Partner
(86) Internationale Anmeldenummer: EP8800690
(87) Internationale Veröffentlichungsnummer: WO8901318

(56) Entgegenhaltungen:
- EP-A- 0 085 484
- FR-A- 2 393 623
- US-A- 2 759 265
- US-A- 4 120 090
- US-A- 4 626 209
- Lexikon der Fertigungstechnik und Arbeitsmaschinen, Bd.8, Seiten 200,201
- Laser Optronic, Vortrag vom 06.12.1978 von Dr. J. Buchholz, "Anwendung des Laser-Abtragens in der Feinbearbeitung", Seiten 9,10,22,28-32

## Beschreibung

Die Erfindung betrifft ein metallisches orthodontisches Hilfsteil mit einer optisch sichtbaren Markierungsfläche.

Ferner betrifft die Erfindung ein Verfahren zum Aufbringen oberflächlicher, optisch sichtbarer Markierungsflächen auf metallischen orthodontischen Hilfsteilen.

Bei metallischen orthondontischen Hilfsteilen, wie z.B. Brackets, Bändern, Bukkalröhrchen etc., ist es erforderlich, Markierungen aufzubringen, um deren Orientierung und Zuordnung zu einzelnen Zahntypen eindeutig zu kennzeichnen.

Bislang ist es bekannt, derartige orthodontische Hilfsteile dadurch zu markieren, daß im Bereich der Markierungsfläche zum Beispiel eine Farbschicht aufgetragen wird oder eine mechanische Kennzeichnung, z.B. in Form einer Einkerbung, eingebracht wird.

Die orthodontischen Hilfsteile können nach einer Verwendung durch Recycling-Verfahren wieder verwendbar gemacht werden. Bei diesen Recycling-Verfahren wird zum einen der Kleber, mit dem diese an den Zähnen befestigt waren, zum Beispiel pyrolitisch abgebrannt und zum anderen wird das ganze orthodontische Hilfsteil zum Beispiel einer elektrolytischen Oberflächenbehandlung unterzogen, um die ursprüngliche glänzende Oberfläche wieder herzustellen.

Wenn nun orthodontische Hilfsteile mit Farbmarkierungen einem derartigen Recycling-Verfahren unterzogen werden, so sind diese Farbmarkierungen später nicht mehr sichtbar. Dies hat zwar den Vorteil, daß sich damit nachweisen läßt, daß dieses Hilfsteil einem Recycling-Verfahren unterzogen wird, allerdings auch den Nachteil, daß dieses orthodontische Hilfsteil dann keinerlei Kennzeichnung mehr tragt und damit nicht mehr eindeutig identifiziert werden kann.

Die Markierungen in Form von mechanisch eingebrachten Kennzeichnungen überdauern zwar eines oder auch mehrere Recycling-Verfahren, sie erlauben jedoch nicht, festzustellen, ob das Jeweilige orthodontische Hilfsteil einem Recycling-Verfahren unterzogen wurde oder nicht.

Ferner haben die Markierungen in Form mechanisch eingebrachter Kennzeichnungen den Nachteil, daß sie im Vergleich zu Farbmarkierungen einen großen maschinellen Aufwand beim Aufbringen erfordern.

Dies trifft insbesondere für die aus der US-A-4120090 bekannten Markierungen zu, bei denen zunächst auf einer blanken Metall(Stahl-)-Oberfläche ein Markierungshintergrund aufgedruckt wird, auf welchen danach die eigentliche Markierung zur Charakterisierung der Größe des orthodontischen Hilfsteils aufgetragen wird.

Der Erfindung liegt daher die Aufgabe zugrunde, ein orthodontisches Hilfsteil der gattungsgemäßen Art zu schaffen, welches selbst einen Nachweis darüber liefert, ob es einer definierbaren Zahl von Recycling-Verfahrenszylden unterzogen wurde.

Diese Aufgabe wird durch ein orthodontisches Hilfsteil der eingangs beschriebenen Art erfindungsgemäß dadurch gelöst, daß die Markierungsfläche eine Oberfläche einer zusätzlich eingebrachten Schmelzgefügeschicht ist, welche eine auf eine vorgebbare Anzahl von durchzuführenden Recycling-Verfahrenszyklen abgestimmten Dicke aufweist.

Unter einer Schmelzgefügeschicht im Sinne der erfindungsgemässen Lösung ist eine Schicht in dem metallischen Hilfsteil zu verstehen, die durch Anschmelzen eines Oberflächenbereichs eines gegossenen oder umgeformten oder sonstwie hergestellten metallischen Hilfsteils entsteht und denen metallographisches Gefüge sich vom Gefüge des ursprünglichen metallischen Hilfsteils unterscheidet.

Eine derartige Schmelzgefügeschicht hat den Vorteil, daß sie sich bei den einzelnen Recycling-Verfahrenszyklen nicht ablöst, sondern im wesentlichen in gleichem Maße wie die übrige Oberfläche des orthodontischen Hilfsteils abgetragen wird. Andererseits läßt sich eine derartige Schmelzgefügeschicht mit einer definierten Dicke anbringen, so daß durch die Dicke der Schmelzgefügeschicht festlegbar ist, wie vielen Recycling-Verfahrenszyklen diese Schicht standhalten soll.

Besonders im Hinblick auf die Einfachheit der Herstellung ist es vorteilhaft, wenn die Schmelzgefügeschicht aus dem Werkstoff des orthodontischen Hilfsteils gebildet ist, so daß sich das Auftragen eines zusätzlichen Werkstoffs erübrigt und lediglich das orthodontische Hilfsteil seinerseits angeschmolzen werden muß.

Um den optischen Unterschied zwischen den übrigen Oberflächenbereichen des orthodontischen Hilfsteils besonders ausgeprägt zu gestalten, hat es sich als nützlich erwiesen, wenn die Schmelzgefügeschicht oberflächlich aufgerauht ist, wobei insbesondere daran gedacht ist, daß die Schmelzgefügeschicht eine kraterähnliche Oberfläche zeigt.

Ferner ist ein deutlicher optischer Unterschied gegenüber den übrigen Oberflächenbereichen des orthodontischen Hilfsteils dadurch erreichbar, daß die Schmelzgefügeschicht Reaktionsprodukte des Metalls mit einem Umgebungsmedium aufweist. Im einfachsten Fall handelt es sich bei diesen Reaktionsprodukten um Metalloxide, die dadurch entstehen, daß die Schmelzgefügeschicht im Beisein von Luft erzeugt wird. Andererseits ist es aber auch möglich, die Schmelzgefügeschicht im Beisein anderer Medien zu erzeugen und dadurch eine noch charakteristischere Anlauffarbe, bedingt durch die jeweiligen Reaktionsprodukte, zu erreichen.

Wie bereits im Vorstehenden ausgeführt, bietet die Schmelzgefügeschicht die Möglichkeit, durch Variation der Dicke die Zahl der Recycling-Verfahrenszyklen festzulegen, welchen die Markierung standhält.

So ist im einfachsten Fall vorgesehen, daß die Schmelzgefügeschicht eine konstante Dicke, d.h. Eindringtiefe, in den jeweiligen Oberflächenbereich des orthodontischen Hilfsteils, aufweist. In diesem Fall ist allein durch die Dicke bestimmbar, nach wie vielen Recycling-Verfahrenszyklen die Schmelzgefügeschicht abgetragen ist.

Es ist aber ebenfalls möglich, daß die Schmelzgefügeschicht eine definierte variable Dicke aufweist, so daß beispielsweise nach jedem Verfahrenszyklus die Markierungsfläche durch abgetragene Teile der Schmelzgefügeschicht kleiner wird und damit nachweisbar wird, wie viele Verfahrenszyklen ein derartiges orthodontisches Hilfsteil bereits durchlaufen hat.

Weiterhin ist es ebenfalls möglich, daß die Schmelzgefügeschicht eine stufenförmig variierende Dicke aufweist, so daß die Markierungsfläche nach z.B. einem Recycling-Verfahrenszyklus um einen vorher bestimmbaren Bereich kleiner wird.

Bei all diesen Ausführungsformen, betreffend die Dicke der Schmelzgefügeschicht ist im Rahmen der erfindungsgemäßen Lösung vorgesehen, daß die Dicke ein Mehrfaches, insbesondere ein ganzzahliges Vielfaches, einer bei einem Recycling-Verfahrenszyklus von der Oberfläche des orthodontischen Hilfsteils abgetragenen Materialschicht beträgt.

Ferner liegt der Erfindung die Aufgabe zugrunde, ein möglichst einfaches Verfahren zur Herstellung eines metallischen orthodontischen Hilfsteils mit optisch sichtbarer Markierungsfläche zu schaffen, so daß das erzeugte Hilfsteil selbst einen Nachweis darüber liefert, ob es einer definierbaren Zahl von Recycling-Verfahrenszyklen unterzogen wurde.

Diese Aufgabe wird bei einem Verfahren der eingangs beschriebenen Art erfindungsgemäß dadurch gelöst, daß im Bereich der Markierungsfläche eine Schmelzgefügeschicht mit einer auf eine vorgebbare Anzahl von durchzuführenden Recycling-Verfahrenszyklen abgestimmten Dicke erzeugt wird.

Diese Schmelzgefügeschicht könnte beispielsweise dadurch erzeugtwerden, daß vor einem Anschmelzen ein zusätslicher Werkstoff aufgetragen wird, der dann sich durch Anschmelzen mit einer Oberfläche des orthodontischen Hilfsteils verbindet. Wesentlich einfacher ist es jedoch, wenn die Schmelzgefügeschicht durch oberflächliches Anschmelzen des Hilfsteils erzeugt wird, so daß kein zusätzlicher Werkstoffauftrag erforderlich ist und das Hilfsteil mit beispielsweise polierten Oberflächen in dem Bereich, in dem die Markierungsfläche angebracht werden soll, lediglich angeschmolzen wird.

Insbesondere zur Verbesserung des optischen Unterschieds einer derartigen Schmelzgefügeschicht gegenüber der übrigen Oberfläche des Hilfsteils ist es vorteilhaft, wenn die Schmelzgefügeschicht durch punktuell unterschiedlich starkes Anschmelzen erzeugt wird, so daß die Oberflächenrauhigkeit im Bereich der Anschmelzung noch verstärkt wird.

Bei dem erfindungsgemäßen Verfahren hat es sich als besonders vorteilhaft erwiesen, wenn die Schmelzgefügeschicht mittels eines Laserstrahls oder eines Elektronenstrahls oder eines Mikroplasmas erzeugt wird, da sich mit diesen in einfacher Weise alle möglichen Formen von Markierungen herstellen lassen und außerdem diese am besten dazu geeignet sind, Schmelzgefügeschichten mit definierten Dikken herzustellen.

Aus der EP-A-0085484 ist zwar bereits prinzipiell das Einbringen von Schmelzgefügeschichten in Metalloberflächen zu Markierungszwekken mittels Laserstrahl bekannt, jedoch ist dort die Zielrichtung eine möglichst dauerhafte, witterungsbeständige Markierung von Stahlteilen in Stahlwerken zu erreichen. Daß eine mittels Laserstrahl eingebrachte Schmelzgefügeschicht einerseits dauerhaft aufgebracht werden kann, daß sie beim Gebrauch der orthodontischen Hilfsteile im wesentlichen unverändert bestehen bleibt, andererseits jedoch unter Recycling-Bedingungen so definiert abbaubar ist, daß sich hieraus eine Kontrolle der Zahl der Recycling-Zyklen ableiten läßt, kann dieser Druckschrift nicht entnommen werden.

Zur Erreichung eines möglichst deutlich sichtbaren optischen Unterschiedes zwischen einer polierten Oberfläche des orthodontischen Hilfsteils und der Schmelzgefügeschicht hat es sich als vorteilhaft erwiesen, wenn die Schmelzgefügeschicht in Gegenwart eines mit dem Metall reagierenden Mediums erzeugt wird, so daß dieser durch entsprechende Wahl des Mediums noch eine besondere Farbe verliehen werden kann. Die einfachste Ausführung dieses Verfahrens sieht vor, daß die Schmelzgefügeschicht in Gegenwart von Luft erzeugt wird, so daß das Metall mit der Luft reagiert und die Reaktionsprodukte die vom Glühen her bekannten typischen Anlauffarben zeigen.

Wie bereits im Zusammenhang mit den Vorteilen des erfindungsgemäßen Verfahrens ausgeführt wurde, gibt dieses die Möglichkeit, Markierungen zu schaffen, die bei den einzelnen Recycling-Verfahrenszyklen nicht stärker abgetragen werden als die übrige Oberfläche des orthodontischen Hilfsteils. Aus diesem Grund ist es im Rahmen der Erfindung vorteilhaft, wenn die Schmelzgefügeschicht mit einer Dicke erzeugt wird, die ein Mehrfaches, insbesondere ein ganzzahliges Vielfaches, der bei einem Recycling-Verfahrenszyklus abgetragenen Schichtdicke beträgt.

Somit besteht zum Beispiel die Möglichkeit, bei einer Schmelzgefügeschicht mit konstanter Dicke diese so zu wählen, daß sie nach einem oder nach mehreren Recycling-Verfahrenszyklen vollkommen abgetragen und damit nicht mehr sichtbar ist.

Es besteht bei einer Schmelzgefügeschicht mit unterschiedlicher Dicke aber auch die Möglichkeit, daß sich die Markierungsfläche nach jedem Recycling-Verfahrenszyklus entsprechend dem vollständig abgetragenen Teil der Schmelzgefügeschicht verkleinert, so daß dadurch jeder einzelne Markierungszyklus nachweisbar ist. Besonders vorteilhaft ist es in diesem Zusammenhang, wenn die Schmelzgefügeschicht mit stufenförmig zunehmender Dicke erzeugt wird, wobei die Dickenänderung bei einer Stufe der bei einem Recycling-Verfahrenszyklus abgetragenen Schichtdicke entspricht, so daß die Markierungsfläche nach jedem Recyclings-Verfahrenszyklus stufenweise kleiner wird.

Das erfindungsgemäße Verfahren ist aber auch vorteilhaft dazu anwendbar, eine Markierung zu erzeugen, die sämtlichen Recyclings-Verfahrenszyklen, die mit einem orthodontischen Hilfsteil im Rahmen seiner Lebensdauer durchführbar sind, standhält. Auch hierbei wirkt sich der Vorteil, daß mit dem erfindungsgemäßen Verfahren Schmelzgefügeschichten mit definierter Dicke erzeugbar sind, positiv aus, da in diesem Fall die Dicke der Schmelzgefügeschicht so gewählt werdem kann, daß sie in allen Bereichen größer ist als die Summe der bei der Lebensdauer eines derartigen Hilfsteils durch Recycling-Verfahrenszyklen abgetragenen Schichtdicken, so daß die Markierungsfläche nach jedem Recycling-Verfahrenszyklus noch unverändert dieselbe Größe hat.

Weitere Merkmale und Vorteile der Erfindung sind Gegenstand der nachfolgenden Beschreibung sowie der zeichnerischen Darstellung einiger Ausführungsbeispiele. In der Zeichnung zeigen :
- Fig. 1: eine perspektivische Ansicht eines ersten Ausführungsbeispiels eines Hilfsteils ;
- Fig. 2: einen Schnitt längs Linie 2-2 in Fig. 1 und
- Fig. 3: einen Schnitt ähnlich Fig. 2 bei einem zweiten Ausführungsbeispiel.

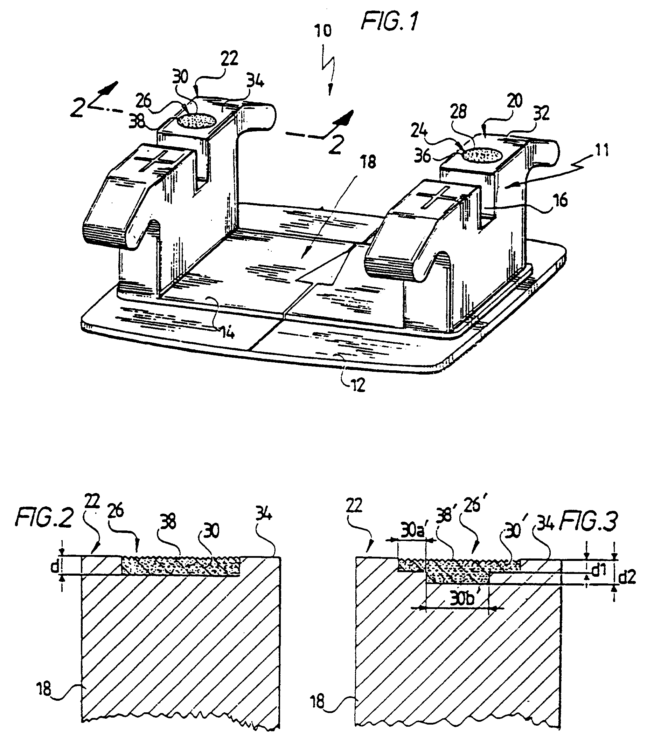

Ein als Ganzes mit 10 bezeichnetes erstes Ausführungsbeispiel eines erfindungsgemäßen Hilfsteils in Fig. 1 umfaßt ein Bracket 11, dessen Grundfläche mit einer Retentionsbasis 12 verschweißt ist. Das Bracket 11 ist üblicherweise profiliert, wobei eine Porfilart, wie aus Fig. 1 ersichtlich, typisch ist. In ein solches Bracket-Profil werden üblicherweise Längs- 16 und Quernuten 18 eingebracht. Dadurch entstehen kleine Bereiche auf den der Basisfläche 14 abgewandten Frontseiten 20/22, auf denen Markierungen 24, 26 angebracht sind. Ebenso sind Markierungen im Bereich der Quernut 18 möglich.

Diese punktförmigen Markierungen 24 bzw. 26 sind, wie in Fig. 2 im Schnitt dargestellt, Schmelzgefügeschichten 28 bzw. 30, die sich von einer Oberfläche 32 bzw. 34 der jeweiligen Frontseite 20 bzw. 22 aus in Richtung der Retentionsbasis 12 in das Bracket 11 mit einer Dicke d hinein erstrecken. Im Bereich dieser Schmelzgefügeschichten 28 bzw. 30 wurde das Metall, aus welchem das Bracket 11 hergestellt ist, mittels eines Laserstrahls über den Schmelzpunkt erhitzt, so daß das Gefüge, beispielsweise bei Erhitzung in Luft, die für das geglühte Metall charakteristische Anlauffarbe zeigt, welche durch chemische Reaktion des über dem Schmelzpunkt erhitzten Metalls mit der Luft entsteht. Ferner ist eine Oberfläche 36 bzw. 38 der Schmelzgefügeschicht 28 bzw. 30 nicht mehr glatt poliert wie die Oberflächen 32 bzw. 34 der Frontseiten 20 bzw. 22, sondern rauh, was beispielsweise dadurch erreichbar ist, daß der Laserstrahl beim Herstellen der Schmelzgefügeschichten 28 bzw. 30 rasterartig Ober die Oberfläche gefahren wird.

Die Dicke d der Schmelzgefügeschicht 28 bzw. 30 hängt ab von der Energie, die punktförmig dem jeweils von dem Laserstrahl getroffenen Oberflächenbereich zugeführt wird, so daß bei größerer Energie des Laserstrahls oder Verweilzeit des Laserstrahls auf dem jeweiligen Punkt eine größere Dicke d der Schmelzgefügeschicht erreichbar ist und letztere daher sich weiter ins Innere des jeweiligen Bracketflügels 16 bzw. 18 hinein erstreckt.

Bei dem in Fig. 2 dargestellten Ausführungsbeispiel ist die Dicke d quer über die gesamte Schmelzgefügeschicht 28 bzw. 30 konstant gewählt, so daß sich bei einem oberflächlichen Abtrag der Schmelzgefügeschicht 28 bzw. 30 im Zusammenhang mit einem Recycling-Verfahrenszyklus die jeweils sichtbare Oberfläche 36 bzw. 38 der jeweiligen Markierung 24 bzw. 26 in ihrer Ausdehnung nicht ändert.

Im Gegensatz dazu zeigt das zweite Ausführungsbeispiel, dargestellt in Fig. 3, eine Markierung 26' mit stufenförmigem Querschnitt der Schmelzgefügeschicht 30'. Diese Schmelzgefügeschicht 30' ist unterteilt in einen äußeren Bereich 30a' mit der Dicke d1 und einen inneren Bereich 30b' mit der Dicke d2, die beispielsweise ein Vielfaches der Dicke d1 beträgt.

Wird bei diesem Ausführungsbeispiel die Schmelzgefügeschicht 30', beispielsweise nach mehreren Recycling-Verfahrenszyklen, um die Schichtdicke d1 abgetragen, so verändert sich die Größe der Oberfläche 38', da durch den Abtrag die äußeren Bereiche 30a' der Schmelzgefügeschicht abgetragen sind und nur noch die inneren Bereiche 30b' mit einer Dicke d2 minus d1 stehen geblieben sind. Somit ist die Markierung 26' zwar noch vorhanden, zeigt aber eine kleinere Oberfläche entsprechend dem inneren Bereich 30b'.

Die Dicke d1 der Schmelzgefügeschicht 30' kann nun so gewähltwerden, daß sie beispielsweise nach zwei Recycling-Verfahrenszyklen abgetragen ist, so daß sich sofort an der Größe der Oberfläche 38' ablesen läßt, wievielen Recycling-Verfahrenszyklen das jeweilige Bracket unterworfen war.

Erfindungsgemäß werden die Dicken d, d1, d2 der Schmelzgefügeschichten 30, 30' im Bereich von ungefähr 0,1 µm bis 0,5 µm gewählt, wobei eine Schmelzgefügeschicht 30, 30' mit einer Dicke d, d1, d2 von 0,1 µm in der Regel ungefähr einem Recycling-Verfahrenszyklus standhält und nach einem solchen abgetragen ist Dagegen kann eine Schmelzgefügeschicht 30, 30' mit einer Dicke d, d1, d2 von ungefähr 0,5 µm als recyclinbeständig bezeichnet werden, da eine solche erst nach ungefähr 5 Recyclings-Verfahrenszyklen abgetragen wäre und bei einem Bracket in der Regel fünf Recyclings-Verfahrenszyklen nicht denkbar sind.

Die vorstehende Beschreibung der Markierung eines erfindungsgemäßen Brackets trifft in gleicher Weise auf die Markierungen von Bändern und Bukkalröhrchen zu.

## Patentansprüche

1. Metallisches orthodontisches Hilfsteil mit einer optisch sichtbaren Markierungsfläche, dadurch gekennzeichnet, daß die Markierungsfläche eine Oberfläche (36, 38) einer zusätzlich eingebrachten Schmelzgefügeschicht (26, 28, 30) ist, welche eine auf eine vorgebbare Anzahl von durchzuführenden Recycling-Verfahrenszyklen abgestimmte Dicke aufweist.

2. Metallisches orthodontisches Hilfsteil nach Anspruch 1, dadurch gekennzeichnet, daß die Schmelzgefügeschicht (28, 30) aus dem Werkstoff des orthodontlschen Hilfsteils gebildet ist.

3. Metallisches orthodontisches Hilfsteil nach einem der Ansprüche 1 oder 2, dadurch gekennzeichnet, daß die Schmelzgefügeschicht (28, 30) oberflächlich aufgerauht ist.

4. Metallisches orthodontisches Hilfsteil nach einem der voranstehenden Ansprüche, dadurch gekennzeichnet, daß die Schmelzgefügeschicht (28, 30) Reaktionsprodukte des Werkstoffs mit einem bei deren Herstellung vorhandenen Umgebungsmedium aufweist.

5. Metallisches orthodontisches Hilfsteil nach einem der voranstehenden Ansprüche, dadurch gekennzeichnet, daß die Schmelzgefügeschicht (28, 30) eine konstante Dicke (d) aufweist.

6. Metallisches orthodontisches Hilfsteil nach einem der Ansprüche 1 bis 4, dadurch gekennzeichnet, daß die Schmelzgefügeschicht (30') eine variable Dicke (d1, d2) aufweist.

7. Metallisches orthodontisches Hilfsteil nach Anspruch 6, dadurch gekennzeichnet, daß die Schmelzgefügeschicht (30') eine stufenförmige variierande Dicke (d1, d2) aufweist.

8. Verfahren zum Herstellen eines metallischen, orthodontischen Hilfsteils mit oberflächlicher, optisch sichtbarer Markierungsfläche, insbesondere zur Herstellung eines orthodontischen Hilfsteils nach einem der Ansprüche 1 bis 7, dadurch gekennzeichnet, daß im Bereich der Markierungsfläche eine Schmelzgefügeschicht mit einer auf eine vorgebbare Anzahl von durchzuführenden Recycling-Verfahrenszyklen abgestimmten Dicke erzeugt wird.

9. Verfahren nach Anspruch 8, dadurch gekennzeichnet, daß die Schmelzgefügeschicht durch oberflächliches Anschmelzen des Hilfsteils erzeugt wird.

10. Verfahren nach Anspruch 8 oder 9, dadurch gekennzeichnet, daß die Schmelzgefügeschicht durch punktuell unterschiedlich starkes Anschmelzen erzeugt wird.

11. Verfahren nach einem der Ansprüche 8 bis 10, dadurch gekennzeichnet, daß die Schmelzgefügeschicht mittels eines Laserstrahls erzeugt wird.

12. Verfahren nach einem der Ansprüche 8 bis 10, dadurch gekennzeichnet, daß die Schmelzgefügeschicht mittels eines Elektronenstrahls erzeugt wird.

13. Verfahren nach einem der Ansprüche 8 bis 10, dadurch gekennzeichnet, daß die Schmelzgefügeschicht mittels eines Mikroplasmas erzeugt wird.

14. Verfahren nach einem der Ansprüche 8 bis 13, dadurch gekennzeichnet, daß die Schmelzgefügeschicht in Gegenwart eines mit dem Metall reagierenden Mediums erzeugt wird.

15. Verfahren nach einem der Ansprüche 8 bis 14, dadurch gekennzeichnet, daß die Schmelzgefügeschicht mit konstanter Dicke erzeugt wird.

16. Verfahren nach einem der Ansprüche 8 bis 14, dadurch gekennzeichnet, daß die Schmelzgefügeschicht in unterschiedlicher Dicke erzeugt wird.

17. Verfahren nach einem Anspruch 16, dadurch gekennzeichnet, daß die Schmelzgefügeschicht mit im Querschnitt stufenförmig variierender Dicke erzeugt wird.

## Claims

1. Metallic orthodontic aid, having an optically visible marking area, characterized in that the marking area is a surface (36, 38) of an additionally produced layer with a remelt structure (26, 28, 30) which has a thickness adapted to a predeterminable number of recycling process cycles to be carried out.

2. Metallic orthodontic aid according to Claim 1, characterized in that the layer with a remelt structure (28, 30) is formed from the material of the orthodontic aid.

3. Metallic orthodontic aid according to one of Claims 1 or 2, characterized in that the layer with a remelt structure (28, 30) is roughened at the surface.

4. Metallic orthodontic aid according to one of the preceding claims, characterized in that the layer with a remelt structure (28, 30) has reaction products of the material with an ambient medium available during its manufacture.

5. Metallic orthodontic aid according to one of the preceding claims, characterized in that the layer with a remelt structure (28, 30) has a constant thickness (d).

6. Metallic orthodontic aid according to one of Claims 1 to 4, characterized in that the layer with a remelt structure (30') has a variable thickness (d1, d2).

7. Metallic orthodontic aid according to Claim 6, characterized in that the layer with a remelt structure (30') has a step-wise varying thickness (d1, d2).

8. Process for manufacturing a metallic, orthodontic aid with a superficial, optically visible marking area, in particular for the manufacture of an orthodontic aid according to one of Claims 1 to 7, characterized in that there is produced in the region of the marking area a layer with a remelt structure with a thickness adapted to a predeterminable number of recycling process cycles to be carried out.

9. Process according to Claim 8, characterized in that the layer with a remelt structure is produced by surface melting of the aid.

10. Process according to Claim 8 or 9, characterized in that the layer with a remelt structure is produced by surface melting at an intensity varying from point to point.

11. Process according to one of Claims 8 to 10, characterized in that the layer with a remelt structure is produced by means of a laser beam.

12. Process according to one of Claims 8 to 10, characterized in that the layer with a remelt structure is produced by means of an electron beam.

13. Process according to one of Claims 8 to 10, characterized in that the layer with a remelt structure is produced by means of a microplasma.

14. Process according to one of Claims 8 to 13, characterized in that the layer with a remelt structure is produced in the presence of a medium reacting with the metal.

15. Process according to one of Claims 8 to 14, characterized in that the layer with a remelt structure is produced with a constant thickness.

16. Process according to one of Claims 8 to 14, characterized in that the layer with a remelt structure is produced with varying thickness.

17. Process according to Claim 16, characterized in that the layer with a remelt structure is produced with a thickness varying step-wise in cross-section.

## Revendications

1. Accessoire orthodontique métallique présentant une surface de repérage optiquement visible, caractérisé en ce que la surface de repérage est une surface (36, 38) d'une couche structurale fondue (26, 28, 30) introduite additionnellement et qui présente une épaisseur définie en fonction d'un nombre préalablement déterminable de cycles d'opérations de recyclage à exécuter.

2. Accessoire orthodontique métallique selon la revendication 1, caractérisé en ce que la couche structurale fondue (28, 30) est formée du matériau de l'accessoire orthodontique.

3. Accessoire orthodontique métallique selon l'une des revendications 1 ou 2, caractérisé en ce qu'est rendue superficiellement rugueuse la couche structurale fondue (28, 30).

4. Accessoire orthodontique métallique selon l'une des revendications précédentes, caractérisé en ce que la couche structurale fondue (28, 30) présente des produits de la réaction du matériau avec un milieu ambiant présent au cours de sa production.

5. Accessoire orthodontique métallique selon l'une des revendications précédentes, caractérisé en ce que la couche structurale fondue (28, 30) présente une épaisseur constante (d).

6. Accessoire orthodontique métallique selon l'une des revendications 1 à 4, caractérisé en ce que la couche structurale fondue (30') présente une épaisseur variable (d1, d2).

7. Accessoire orthodontique métallique selon la revendication 6, caractérisé en ce que la couche structurale fondue (30') présente une épaisseur (d1, d2) variant par paliers.

8. Procédé de production d'un accessoire orthodontique métallique présentant une surface de repérage superficielle, optiquement visible, notamment de production d'un accessoire orthodontique selon l'une des revendications 1 à 7, caractérisé en ce qu'est produite dans la zone de la surface de repérage une couche structurale fondue d'épaisseur déterminée en fonction d'un nombre préalablement défini de cycles d'opérations de recyclage à exécuter.

9. Procédé selon la revendication 8, caractérisé en ce que la couche structurale fondue est produite par fusion superficielle de l'accessoire.

10. Procédé selon la revendication 8 ou 9, caractérisé en ce que la couche structurale fondue est produite par fusion d'intensité ponctuellement différente.

11. Procédé selon l'une des revendications 8 à 10, caractérisé en ce que la couche structurale fondue est produite au moyen d'un rayon laser.

12. Procédé selon l'une des revendications 8 à 10, caractérisé en ce que la couche structurale fondue est produite au moyen d'un faisceau électronique.

13. Procédé selon l'une des revendications 8 à 10, caractérisé en ce que la couche structurale fondue est produite au moyen d'un microplasma.

14. Procédé selon l'une des revendications 8 à 13, caractérisé en ce que la couche structurale fondue est produite en présence d'un milieu réagissant avec le métal.

15. Procédé selon l'une des revendications 8 à 14, caractérisé en ce que la couche structurale fondue est produite en une épaisseur constante.

16. Procédé selon l'une des revendications 8 à 14, caractérisé en ce que la couche structurale fondue est produite en une épaisseur différente.

17. Procédé selon la revendication 16, caractérisé en ce que la couche structurale fondue est produite en une épaisseur variant par paliers dans sa section transversale.
